Europäisches Patentamt

(19) European Patent Office

Office européen des brevets

(11) Numéro de publication: **0 037 407**
**B1**

(12) **FASCICULE DE BREVET EUROPEEN**

(45) Date de publication du fascicule du brevet: **22.02.84**  (51) Int. Cl.³: **A 61 K 9/36,** A 61 K 9/28

(21) Numéro de dépôt: **80901943.3**

(22) Date de dépôt: **16.10.80**

(86) Numéro de dépôt international:
PCT/FR80/00151

(87) Numéro de publication internationale:
WO 81/01100 30.04.81 Gazette 81/11

(54) PROCEDE DE DRAGEIFICATION DURE AU SORBITOL.

(30) Priorité: **17.10.79 FR 7925840**
**03.04.80 CH 2652/80**

(43) Date de publication de la demande:
**14.10.81 Bulletin 81/41**

(45) Mention de la délivrance du brevet:
**22.02.84 Bulletin 84/8**

(84) Etats contractants désignés:
**DE GB NL SE**

(56) Documents cités:
**DE - B - 2 636 152**
**FR - A - 1 137 104**
**FR - A - 2 108 470**
**GB - A - 1 123 336**
**US - A - 2 841 528**
**US - A - 2 925 365**
**US - A - 3 185 626**
**US - A - 3 361 631**

(73) Titulaire: **Roquette Frères**
**F-62136 Lestrem (FR)**

(72) Inventeur: **DEVOS, Francis**
**Route de Merville Morbecque**
**F-59660 Merville (FR)**
Inventeur: **BUSSIERE, Guy**
**64, rue de la Lys**
**F-59253 La Gorgue (FR)**
Inventeur: **HUCHETTE, Michel**
**63, rue du Maréchal Joffre**
**F-59660 Merville (FR)**

(74) Mandataire: **Koch, Gustave et al,**
**Cabinet PLASSERAUD 84, rue d'Amsterdam**
**F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# 0 037 407

## Procédé de dragéification dure au sorbitol

L'invention a pour objet un procédé de dragéification dure.

On rappelle que, par l'expression "dragéification dure", on désigne tout procédé consistant à enrober un élément préformé ou noyau d'une enveloppe adhérente:

— protégeant le noyau contre les agents physicochimiques extérieurs dont l'humidité atmosphérique et l'oxygène de l'air,

— conférant à l'article fini une plus grande résistance méchanique contre les chocs et l'abrasion ainsi qu'un aspect plus agréable et une saveur ou un goût particulier.

Le concept de la dragéification dite "dure" se distingue de celui de la dragéification dite "tendre" en ce qu'il sous-entend une cristallisation complète de l'enveloppe avec évaporation de la totalité de l'eau contenue dans la matière constitutive de celle-ci.

C'est dans l'industrie de la confiserie et des produits pharmaceutiques que la dragéification dure trouve ses principales applications, étant rappelé que les "confiseries" susceptibles d'être dragéifiées comprennent notamment les chewing-gums, pâtes à mâcher, bonbons et réglisses, et que les produits pharmaceutiques, susceptibles de dragéification, sont constitués notamment par les tablettes, comprimés ou bonbons comportant des substances médicamenteuses ou principes actifs.

Tant les confiseries que les produits pharmaceutiques susmentionnés peuvent être de type traditionnel, c'est-à-dire comportant des sucres tels que le saccharose, le dextrose, le fructose ou les sirops de glucose; ils peuvent également être du type "sans sucre", c'est-à-dire à base notamment de polyols tels que le sorbitol, le xylitol, le mannitol ou l'hydrolysat hydrogèné non cariogène connu sous la marque LYCASIN®, et dépourvus de saccharose, dextrose, fructose, sirops de glucose ou produits équivalents.

L'enrobage du noyau est effectué dans une cuve inclinée tournant autour de son axe et appelée drageuse, à l'intérieur de laquelle se trouvent une pluralité de noyaux formant une masse en mouvement, à la surface de laquelle on répartit à l'état liquide la matière constitutive de la future enveloppe.

Cette matière constitutive est traditionellement du saccharose.

Or, le saccharose, comme d'autres sucres tels que le dextrose ou le fructose, a des effets biologiques indésirables, notamment du point de vue de la carie dentaire. Il est en effet très facilement et très rapidement acidifiable par les bactéries de la bouche. Par ailleurs, la saveur sucrée du saccharose est souvent peu appréciée, surtout en saison chaude, le consommateur recherchant alors, dans les chewing-gums et les confiseries en général, une saveur rafraîchissante et moins sucrée.

C'est, bien entendu, surtout quand le noyau est "sans sucre" que le caractère cariogène du saccharose, lorsque ce dernier est utilisé pour la confection de l'enrobage, devient rédhibitoire.

Il a donc tout naturellement été proposé d'avoir recours dans ce cas, pour constituer l'enveloppe lors du procédé de dragéification, aux polyols déjà utilisés pour la constitution des confiseries ou produits pharmaceutiques "sans sucre" destinés à être dragéfiés.

Il se trouve que seuls le xylitol et, dans une moindre mesure, le mannitol, sont actuellement utilisés dans cette application. Ils possèdent en effet, contrairement au sorbitol, une hydroscopicité peu élevée et de bonnes aptitudes à la cristallisation, celle-ci s'effectuant assez facilement à partir de leurs solutions aqueuses pour donner des cristaux de type bien défini.

Pour arriver, avec le xylitol, à un résultat satisfaisant, il a toutefois été nécessaire de faire appel à l'addition d'auxiliaires de fabrication ou d'addition, tels que diverses matières grasses, divers liants ou agents suspensifs, les enveloppes au xylitol seul s'altérant rapidement au cours du temps en se craquelant, leur surface extérieur, lisse au départ, devenant ridée et la structure interne de l'enveloppe, qui présente au départ un caractère microcristallin, prenant une structure rugueuse, désagréable au palais lors de la mastication; de toute façon, on ne peut plus parler alors d'une dragéification dure au xylitol, analogue à celle réalisée avec le saccharose.

En ce qui concerne le sorbitol dont l'application à l'enrobage de dragées de forme quelconque a été proposée en 1955 sans indication des paramètres expérimentaux par le brevet FR—A—1 137 104, il est, depuis quelques années, réputé complètement inapplicable dans un processus de dragéification dure. Ainsi, il a été indiqué clairement, par exemple dans la demande de brevet FR—A—2 342 668, que l'on ne peut utiliser le sorbitol que comme ingrédient du noyau et non de l'enveloppe, en raison de son caractère hydroscopique. On sait, par ailleurs, que la cristallisation du sorbitol est beaucoup plus difficile que celle du xylitol et du mannitol et que cette difficulté est aggravée par le fait que le sorbitol peut être obtenu sous plusieurs formes cristallines différentes, seule la forme gamme constituant une forme stable.

Un procédé d'enrobage connu par la DE—B 2 636 152 utilise bien du sorbitol comme agent d'enrobage, mais associé à un excès de xylitol et, d'autre part, le sirop d'enrobage n'est pas constitué par une solution aqueuse concentrée de sorbitol et de xylitol, mais par une masse fondue de sorbitol et de xylitol, maintenue entre 85 et 90°C. Il est connu, par ailleurs, que le sorbitol et le xylitol ne peuvent être utilisés de façon satisfaisante dans les procédés de dragéification, et que si, par exemple, on pulvérise sur les noyaux du sorbitol ou du xylitol sous forme d'une solution aqueuse à 50 ou 60%, ainsi qu'il est usuel pour les solutions de saccharose, on obtient es produits cassants, fissurés et irréguliers.

Or, prenant notamment en considération le prix de revient nettement moindre du sorbitol par rapport aux xylitol et mannitol susmentionnés, la société demanderesse, et cela en dépit des opinions définitivement défavorables susmentionnées existant à l'égard de l'utilisation du sorbitol dans les procédés de dragéification, a approfondi ses investigations et a eu le mérite de trouver que, de façon inattendue et surprenante, la dragéification dure au sorbitol était possible sous réserve de respecter un certain nombre de conditions opératoires, les produits dragéifiés alors obtenus satisfaisant aux divers desiderata de la pratique.

En conséquence, le procédé de dragéification dure au sorbitol, conforme à l'invention, est caractérisé par le fait que:

d'une part, le sorbitol est mis en oeuvre par addition, sur un lit en mouvement de noyaux à enrober, d'un sirop de température inférieure à 100°C ayant une concentration en matières sèches comprise entre 60 et 85% en poids, de préférence entre 62 et 80% en poids et, plus préférentiellement encore, entre 65 et 77% en poids, la richesse du sirop en D-sorbitol étant supérieure à 80%, de préférence à 95% et, plus préférentiellement encore, à 99%,

— d'autre part, la température régnant dans le lit en mouvement de noyaux à enrober est maintenue à une valeur inférieure à 55°C, de préférence comprise entre 50 et 10°C et, plus préférentiellement encore, entre 40 et 15°C, l'ensemble de ces conditions étant choisi, à l'intérieur des limites indiquées, de façon telle que, lorsque le sirop de sorbitol arrive au contact des noyaux à enrober, c'est-à-dire à la température maintenue dans le lit en mouvement, il se trouve à un niveau de saturation compris entre 0,65 et 1,25, de préférence entre 0,8 et 1,15.

Dans un mode déréalisation avantageux du susdit procédé, la température du sirop de sorbitol mis en oeuvre est inférieure de préférence à 90°C et, plus préférentiellement encore, à 70°C.

On rappelle que, par l'expression "niveau de saturation", on désigne le rapport, pour une température donnée, de la concentration du sirop exprimé en grammes de sorbitol pour 100 cm³ d'eau, sur la limite de solubilité du sorbitol, à la température donnée, églaement exprimée en grammes de sorbitol pour 100 cm³ d'eau; à toutes fins utiles, on indique ci-après les valeurs de la limite de solubilité du sorbitol pour un certain nombre de températures:

| t °C | Limite de solubilité (en g/100 cm³) |
|------|-------------------------------------|
| 20   | 220                                 |
| 25   | 244                                 |
| 30   | 278                                 |
| 35   | 317                                 |
| 40   | 362                                 |
| 45   | 425                                 |
| 50   | 500                                 |

Les produits de dragéification obtenus à l'aide du procédé conforme à l'invention sont caractérisés par le fait que l'enrobage est à base de sorbitol cristallisé sur toute l'epaisseur dudit enrobage.

L'invention pourra être encore mieux comprise à l'aide du complément de description qui suit et des exemples donnés en rapport avec des modes de réalisation avantageux.

Se proposant, par conséquent, de fabriquer des produits de dragéification, on s'y prend comme suit ou de façon équivalente.

On introduit, dans une cuve tournante de dragéification d'un type conventionnel et équipée de moyens de contrôle de la température intérieure, les noyaux à dragéifier du type confiserie ou produit pharmaceutique, "sans sucre" ou non, et on pulvérise sur la masse de noyaux en mouvement un sirop de sorbitol dont la température est inférieure à 100°C, de préférence à 90°C et plus préférentiellement encore à 70°C.

Le sirop de sorbitol pulvérisé mis en oeuvre présente une concentration en matières sèches comprise entre 60 et 85% en poids, de préférence entre 62 et 80% en poids, et, plus préférentiellement encore, entre 65 et 77% en poids.

La richesse de ce sirop en D-sorbitol est supérieure à 80%, de préférence à 95% et, plus préférentiellement encore, à 99%.

3

La température régnant dans le lit de noyaux en mouvement est maintenue à une valeur inférieure à 55°C, de préférence comprise entre 50 et 10°C et, plus préférentiellement encore, entre 40 et 15°C.

La concentration en matières sèches du sirop de sorbitol, d'une part, et la température du lit de noyaux, d'autre part, sont choisies à l'intérieur des limites sus-indiquées de façon telle que, lorsque le sirop de sorbitol arrive au contact des noyaux à enrober, il se trouve à un niveau de saturation compris entre 0,65 et 1,25, de préférence entre 0,8 et 1,15.

De façon conventionnelle, l'enrobage se fait par cycles successifs comprenant chacun une première phase d'addition du sirop de sorbitol sur le lit de noyaux et une seconde phase pendant laquelle on arrête l'addition tout en maintenant la rotation de la cuve et la température régnant au sein de la masse de noyaux, l'enveloppe dont ont été enrobés les noyaux étant séchée et polie au cours de cette phase.

L'épaisseur de l'enveloppe peut être choisie librement en fonction notamment du noyau à dragéifier ou des effets recherchés.

Dans la pratique, pour réaliser une enveloppe d'enrobage de 1 mm d'épaisseur, il convient de réaliser successivement 15 à 20 additions.

Les moyens pour le maintien de la température au sein de la masse de noyaux en mouvement peuvent être constitués par un dispositif d'insufflation d'air chaud de température contrôlée.

Grâce aux conditions d'enrobage conformes à l'invention, il ne se produit à aucun moment du processus ni une fusion, ni un grossissement irrégulier des cristaux de sorbitol en cours de formation, ce qui conduit en fin d'opération à une surface lisse, dure et brillante, sans phénomène de "pelure d'orange" ou autres irrégularités de surface.

Ces conditions maintiennent en outre une viscosité du sirop de sorbitol telle que l'on réalise une excellente répartition de la phase liquide sur les noyaux en cours de grossissement et donc une cristallisation rapide du sorbitol.

Ces conditions, associées à l'insufflation d'air chaud, permettent l'obtention de cristaux très fins et une excellent dragéification.

La société demanderesse a eu un mérite considérable à constater que ce sont les conditions exposées qui conduisent au résultat recherché; en effet, ces conditions sont tout à fait contraires au principe généralement admis, qui consiste, comme par exemple dans le cas du saccharose, à opérer avec un sursaturation élevée pour favoriser la formation des cristaux; il se trouve que, comme la société demanderesse l'a constaté, des solutions de sorbitol, placées aux mêmes niveaux de sursaturation que ceux couramment utilisés avec le saccharose (S > 1,4), étaient d'une viscosité trop élevée, engendrant deux inconvénients majeurs, à savoir:

— d'une part, une mauvaise répartition du sirop liquide sur les noyaux, pouvant entraîner dans certains cas des collages dans la cuve, et,

— d'autre part et surtout, des retards importants à la cristallisation, conduisant à la formation de surfaces irrégulières incomplètement cristallisées et donc présentant les désavantages essentiels d'une mauvaise stabilité et d'un manque de fraîcheur instantanée, cette dernière caractéristique constituant une propriété intrinsèque de la forme cristalline stable du sorbitol.

Il est possible d'ajouter au sirop de sorbitol à pulvériser divers additifs comme des colorants, des arômes ou des agents améliorant l'état de surface tels que la cire d'abeille.

Parmi les colorants, on peut citer le dioxyde de titane, parmi les arômes, ceux de menthe, orange et citron.

On peut également prévoir des agents liants tels que des gommes végétales et la gélatine ou des substances grasses telles que les mono- et diglycérides. On peut également prévoir d'ajouter au sorbitol d'autres sucres comme le xylitol ou le mannitol.

Parmi les produits constitutifs du noyau à enrober et qui peuvent être choisis dans le groupe des confiseries et produits pharmaceutiques sus-indiqués, on fera, en raison du caractère non cariogène de l'enrobage obtenu conformément à l'invention, une mention particulière aux produits "sans sucre" et, parmi ceux-ci, aux bonbons non cariogènes à base de sirops de glucose hydrogénés de marque LYCASIN®.

Lorsque, au contraire, le noyau à enrober contient un sucre fermentescible, l'enrobage obtenu conformément à l'invention atténue le caractère cariogène de l'ensemble et lui confère de toute façon les qualités inhérentes au sorbitol cristallisé de forme stable, notamment la sensation de fraîcheur.

L'invention a donc une portée générale s'adressant à tous produits pharmaceutiques et confiseries à dragéifier.

D'un point de vue général, ou souligne que les produits de dragéification conformes à l'invention présentent une surface lisse et brillante, essentiellement exempte d'imperfections et cristalline sur toute l'épaisseur de l'enveloppe, qu'ils sont stables, même dans des atmosphères à humidité élevée et qu'ils présentent en outre une sensation de fraîcheur très agréable au moment de la consommation, due à la chaleur de dissolution élevée du sorbitol ainsi qu'à sa haute solubilité à 37°C.

# 0 037 407

A. Drageification d'un chewing-gum de type "sans sucre".
La composition retenue était la suivante:

— Gomme de base type PA—LOJA          25 parties en poids

— NEOSORB® poudre 60 (Pf. 96°C)          50 parties en poids

— LYCASIN® 80/55 à 80% de
— matières sèches contenant des
arômes et des parfums colorants          25 parties en poids.

La gomme de base, préalablement réchauffée à 75°C, a été malaxée dans un pétrin du type KÜSTNER muni d'une circulation d'eau chaude, en présence de la phase liquide (LYCASIN® + parfums et colorants); la phase solide (NEOSORB® poudre) a été ajoutée progressivement par petites quantités.

Après un poudrage au mannitol, la pâte a été laminée et découpée en plaquettes de forme classique.

Ce sont ces plaquettes que l'on a dragéifiées.

Pour ce faire, on a placé 500 g des susdites plaquettes dans une drageuse de laboratoire du type "LILLIPUT" du constructeur FROGERAIS, équipée d'une soufflerie d'air régulée pour maintenir constante la compérature du lit de plaquettes, et d'une sonde thermométrique placée dans ce lit.

Vitesse de rotation de la drageuse: 25—30 tours minute.

Le produit d'enrobage constitué d'un sirop de sorbitol a été maintenu à température constante à l'aide d'un bain-marie thermorégulé.

Le sirop d'enrobage est ajouté par charges successives (cycles) de 20 g de sirop, l'introduction de ces charges se faisant en quelques secondes toutes les dix minutes, le temps séparant la fin d'une introduction de l'introduction suivante étant nécessaire pour obtenir la cristallisation du sorbitol et l'évaporation de l'eau ainsi libérée.

La quantité totale de sirop à ajouter dépend, en particulier, de l'épaisseur souhaitée pour l'enveloppe.

Le sorbitol utilisé pour l'enrobage est constitué par un sirop de glucose hydrogéné connu sous la marque NEOSORB® 70/02, dont la pureté sur matières sèches est de 99%.

## Exemple 1

On a procédé à deux essais de dragéification, la température du lit en cours de dragéification étant maintenue à 30°C, valeur imposée par le ramollissement de la gomme.

La concentration du sirop d'enrobage est de 83% de matières sèches.

Les conditions et résultats de ces essais sont réunis dans le tableau I.

### TABLEAU I

| N° de l'essai | 1 | 2 |
|---|---|---|
| Concentration du sirop d'enrobage | 83% | 83% |
| Température du sirop d'enrobage | 70°C | 40°C |
| Température du lit en mouvement | 30°C | 30°C |
| Nombre de cycles | 5 | 5 |
| Poids de sirop mis en oeuvre | 100 g | 100 g |
| Observations | O O | O O |

Dans ce tableau ainsi que dans deux qui apparaissent dans la suite de la description, les symboles figurant dans la ligne "Observations" caractérisent les résultats des essais concernés. Les significations de ces symboles sont réunies dans le tableau suivant:

5

| médiocre | très médiocre | mauvais | très mauvais | acceptable | moyen | bon | très bon |
|---|---|---|---|---|---|---|---|
| O | O | O | O | + | + | + | + |
| | O | O | O | | + | + | + |
| | | O | O | | | + | + |
| | | | O | | | | + |

La surface des dragées est boursoufflée, il y a une mauvaise répartition du sirop et il se produit des collages dans la drageuse après chaque addition.

On est, en effet, dans ces deux cas, dans des conditions de sursaturation élevée (niveau de saturation: 1,70).

Exemple 2

On a procédé à cinq essais en utilisant un sirop à 70% de matières sèches. Les conditions et résultats sont réunis dans le tableau II.

TABLEAU II

| N° de l'essai | 3 | 4 | 5 | 6 | 7 |
|---|---|---|---|---|---|
| Concentration du sirop | 70% | 70% | 70% | 70% | 70% |
| Température du sirop | 90°C | 70°C | 65°C | 40°C | 40°C |
| Température du lit en mouvement | 30°C | 30°C | 30°C | 30°C | 30°C |
| Nombre de cycles d'enrobage | 14 | 21 | 21 | 15 | 21 |
| Poids total de sirop mis en oeuvre Observations | 280 g<br>+ | 420 g<br>+<br>+ | 420 g<br>+<br>+ | 300 g<br>+<br>+<br>+ | 420 g<br>+<br>+<br>+ |

On remarque qu'à 70% de matières sèches, l'amélioration est très nette. Le niveau de saturation est, pour cette concentration et à cette température, de 0.85.

Il y a toujours un léger collage au moment de chaque cycle, suivi d'une rapide fluidisation de la masse en mouvement.

Les résultats s'améliorent au fur et à mesure que la température d'addition diminue. On souligne la bonne tenue au stockage et l'excellente fraîcher instantanée à la consommation des produits obtenus.

Exemple 3

On a procédé de la manière indiquée à l'exemple 2, la concentration du sirop étant de 75% de matières sèches (niveau de saturation: 1,08).

Les résultats enregistrés sont très bons, la température du sirop d'enrobage n'ayant pas excédé 90°C. On note la bone tenue au stockage des produits obtenus et leur excellent fraîcheur instantanée.

B. Drageification de produits de compression

Les noyaux à dragéifier ont été préparés sur une machine à compression FROGERAIS rotative à 16 poinçons du type MR 2, fonctionnant par compression directe.

Le produit utilisé est le sorbitol poudre de marque NEOSORB® 20/60 aromatisé à la menthe et comportant 0,3% de lubrifiant constitué par du stéarate de magnésium.

La dragéification a été conduite au moyen de l'équipement décrit à propos de la dragéification des chewing-gums de type "sans sucre".

Dans le cas de la dragéification de ces produits de compression au NEOSORB®, la température du lit de dragéification a pu être élevée à 70°C, puisque la température de fusion du NEOSORB® est supérieure à 95°C.

Les additions de sirop d'enrobage ont été de 20 g toutes les 10 minutes.

Exemple 4

A l'aide de neuf essais, on a étudié l'influence de la température du lit en cours de dragéification

# 0 037 407

et de la température d'un sirop d'enrobage, dont la concentration est de 70% de matières sèches. Les conditions de ces essais et les résultats enregistrés sont réunis dans le tableau III.

TABLEAU III

| N° de l'essai | 8 | 9 | 10 | 11 | 12 | 13 | 14 | 15 | 16 |
|---|---|---|---|---|---|---|---|---|---|
| Concentration du sirop (%) | 70 | 70 | 70 | 70 | 70 | 70 | 70 | 70 | 70 |
| Température du sirop (°C) | 90 | 70 | 60 | 90 | 70 | 90 | 80 | 70 | 40 |
| Nombre de cycles | 5 | 12 | 14 | 19 | 21 | 21 | 34 | 21 | 21 |
| Poids de sirop ajouté (en g) | 100 | 240 | 280 | 380 | 420 | 420 | 680 | 420 | 420 |
| Température du lit (°C) | 70 | 70 | 70 | 50 | 40 | 30 | 30 | 30 | 30 |
| Niveau de saturation | <0,40 | <0,40 | <0,40 | 0,56 | 0,67 | 0,85 | 0,85 | 0,85 | 0,85 |
| Observations | O<br>O<br>O<br>O | O<br>O<br>O<br>O | O<br>O<br>O | O<br>O | + | + | +<br>+ | +<br>+<br>+ | +<br>+<br>+<br>+ |

Il apparaît, à l'examen des résultats réunis dans ce tableau III, que, pour un sirop d'enrobage à 70% de matières sèches, on a intérêt à maintenir la température du lit en cours de dragéification en dessous de 40°C. Au-dessus de cette température, la solubilité du sorbitol est très élevée et le sirop à 70% de matières sèches, placé dans ces conditions, est en très forte sous-saturation. A 30°C (essais 13 à 16), on constate une amélioration sensible des résultats au fur et à mesure que la température du sirop d'enrobage diminue, passant de 90 à 40°C.

Pour ces essais, on souligne la bonne cristallinité de la couche, l'excellent stabilité au stockage et la bonne fraîcheur instantanée.

## Exemple 5

On procède d'une façon identique à celle exposée à propos de l'exemple 4, en ayant recours à un sirop d'une concentration de 75% de matières sèches.

Les résultats enregistrés en maintenant à 40°C la température du lit de noyaux en mouvement et, inférieure à 90°C, de préférence inférieure à 70°C, la température du sirop d'enrobage, sont excellents.

## Exemple 6

On a procédé à huit essais de la manière indiquée à l'exemple 4, la concentration du sirop étant de 80%.

Les conditions et résultats sont réunis dans le tableau IV.

TABLEAU IV

| N° de l'essai | 17 | 18 | 19 | 20 | 21 | 22 | 23 | 24 |
|---|---|---|---|---|---|---|---|---|
| Concentration du sirop (%) | 80 | 80 | 80 | 80 | 80 | 80 | 80 | 70 |
| Température du sirop (°C) | 70 | 110 | 80 | 70 | 60 | 110 | 50 | 70 |
| Nombre de cycles | 14 | 14 | 14 | 14 | 14 | 14 | 14 | 14 |
| Poids de sirop ajouté (en g) | 280 | 280 | 280 | 280 | 280 | 280 | 280 | 280 |
| Température du lit (°C) | 70 | 50 | 50 | 50 | 60 | 45 | 45 | 30 |
| Niveau de saturation | <0,50 | 0,80 | 0,80 | 0,80 | 0,80 | 0.95 | 0,95 | 1,43 |
| Observations | O<br>O<br>O | O<br>O | + | +<br>+ | +<br>+ | O<br>O | + | O<br>O |

7

A la lecture de ces résultats, on constate de nouveau que la température du lit en cours de dragéification est un facteur déterminant.

Dans l'essai 17, la température régnant dans le lit, c'est-à-dire 70°C, est trop élevée.

A une température de lit de 50°C, les résultats sont satisfaisants lorsque la température du sirop est de 80, 70 ou 60°C (essais 19, 20 et 21).

Dans le cas de l'essai 18, la température du sirop d'enrobage étant de 110°C, le résultat est mauvais (il se produit apparemment une refonte partielle de la surface du noyau au moment de l'addition de sirop, le sorbitol fondant à 96°C).

Le résultat est satisfaisant pour l'essai 23, la température du lit étant de 45°C.

Pour l'essai 22, les commentaires sont les mêmes que pour l'essai 18.

A l'essai 24, la température du lit étant de 30°C, le sirop d'enrobage devient trop visqueux au moment de l'addition sur le lit en mouvement (niveau de saturation: 1,43), d'où une mauvaise répartition de la pellicule liquide.

C. Drageification de bonbonds "sucre cuit" du type "sans sucre".

Des sucres cuits ont été préparés au laboratoire en évaporant jusqu'à une humidité résiduelle inférieure à 0,5% un hydrolysat d'amidon hydrogéné du type de celui commercialisé sous la marque LYCASIN® 80/55.

Les bonbons ainsi préparés ont été mis sous forme sphérique (diamètre: environ 1,5 cm).

Pour la dragéification, on a utilisé le matériel et les conditions de mise en oeuvre décrits plus haut. La température du lit en mouvement a été maintenue à une valeur inférieure ou au plus égal à 30°C, cette valeur est imposée par la matière constitutive des noyaux.

Toutes les dragéifications ont été effectuées sur 500 g de noyaux.

Les conditions et résultats sont réunis dans le tableau V.

TABLEAU V

| Essai n° | 25 | 26 | 27 | 28 |
|---|---|---|---|---|
| Concentration du sirop (%) | 70 | 70 | 75 | 80 |
| Température du sirop (°C) | 40 | 90 | 80 | 80 |
| Nombre de cycles | 15 | 15 | 15 | 15 |
| Température du lit (°C) | 25 | 25 | 30 | 30 |
| Niveau de saturation | 0,97 | 0,97 | 1,1 | 1,43 |
| Observations | + + + | + + + | + + | O O |

A l'examen des résultats réunis dans ce tableau V, on constate que, pour une température de lit de 25 à 30°C, la dragéification est bonne dans le cas des sirops ayant 70 et 75% de matières sèches. La cristallinité de la surface est bonne, de même que la fraîcheur instantanée (essais 25, 26, 27).

Cette dragéification améliore la tenue au stockage des bonbons au LYCASIN® et leur donne une fraîcheur instantanée agréable.

A 80% de matières sèches (essai 28), le sirop d'enrobage devient visqueux et il se produit des collages importants dans la drageuse, dûs à la surfusion du sorbitol liquide.

**Revendications**

1. Procédé de dragéification dure au sorbitol, caractérisé par le fait que:

— d'une part, le sorbitol est mis en oeuvre par addition, sur un lit en mouvement de noyaux à enrober, d'un sirop de température inférieure à 100°C ayant une concentration en matières sèches comprise entre 60 et 85% en poids, la richesse du sirop en D-sorbitol étant supérieure à 80%,

— d'autre part, la température régnant dans le lit en mouvement de noyaux à enrober est maintenue à une valeur inférieure à 55°C, l'ensemble de ces conditions étant choisi, à l'intérieur des limites indiquées, de façon telle que, lorsque le sirop de sorbitol arrive au contact des noyaux à enrober, c'est-à-dire à la température maintenue dans le lit en mouvement, il se trouve à un niveau de saturation compris entre 0,65 et 1,25.

2. Procédé selon la revendication 1, caractérisé par le fait que la température du sirop de sorbitol mis en oeuvre est inférieure à 90°C.

8

# 0 037 407

3. Procédé selon l'une des revendications 1 et 2, caractérisé par le fait que la température du sirop de sorbitol mis en oeuvre est inférieure à 70°C.

4. Procédé selon l'une des revendications 1 à 3, caractérisé par le fait que le sirop de sorbitol mis en oeuvre présente une concentration en matières sèches comprise entre 62 et 80% en poids.

5. Procédé selon l'une des revendications 1 à 3, caractérisé par le fait que le sirop de sorbitol mis en oeuvre présente une concentration en matières sèches comprise entre 65 et 77% en poids.

6. Procédé selon l'une des revendications 1 à 5, caractérisé par le fait que la richesse en D-sorbitol du sirop de sorbitol mis en oeuvre est supérieure à 95%.

7. Procédé selon l'une des revendications 1 à 6, caractérisé par le fait que la richesse en D-sorbitol du sirop de sorbitol mis en oeuvre est supérieure à 99%.

8. Procédé selon l'une des revendications 1 à 7, caractérisé par le fait que la température du lit en mouvement de noyaux à enrober est maintenue à une valeur comprise entre 50 et 10°C.

9. Procédé selon l'une quelconque des revendications 1 à 7, caractérisé par le fait que la température régnant dans le lit en mouvement de noyaux à enrober est maintenue à une valeur comprise entre 40 et 15°C.

10. Procédé selon l'une quelconque des revendications 1 à 9, caractérisé par le fait que le sorbitol. arrivant au niveau des noyaux à enrober se trouve à un niveau de saturation compris entre 0,8 et 1,15.

11. Application du procédé selon l'une des revendications 1 à 10 à la dragéification de noyaux du domaine de la confiserie et des produits pharmaceutiques.

## Patentansprüche

1. Verfahren zum Hartdragieren mit Sorbit dadurch gekennzeichnet, dass
— einerseits der Sorbit in Anwendung gebracht wird durch Zufügung auf ein bewegtes Bett von zu überziehenden Kernen eines Sirups dessen Temperatur unter 100°C liegt, der einen Trockensubstanzgehalt zwischen 60 und 85 Gew-% aufweise und dessen Gehalt an D-Sorbit über 80% liegt,
— andererseits die innerhalb des bewegten Bettes aus zu überziehenden Kernen herrschende Temperatur auf einem Wert von unter 55°C gehalten wird, wobei diese Bedingungen insgesamt innerhalb der angegebenen Grenzen derartig ausgewählt werden, dass der Sirup sich bei einem Sättigungsniveau zwischen 0,65 und 1,25 befindet, wenn er in Kontakt mit den zu überziehenden Kernen das heist auf die innerhalb des bewegten Betts unterhaltene Temperatur kommt.

2. Verfahren nach Anspruch 1, dadurch gekennzeichnet, dass die Temperatur des in Anwendung gebrachten Sorbitsirups unter 90°C liegt.

3. Verfahren nach einem der Ansprüche 1 und 2, dadurch gekennzeichnet, dass die Temperatur des in Anwendung gebrachten Sorbitsirups unter 70°C liegt.

4. Verfahren nach einem der Ansprüche 1 bis 3, dadurch gekennzeichnet, dass der in Andwendung gebrachte Sorbitsirup einen Trockensubstanzgehalt zwischen 62 und 80 Gew-% aufweist.

5. Verfahren nach einem der Ansprüche 1 bis 3, dadurch gekennzeichnet, dass der in Anwendung gebrachte Sorbitsirup einen Trockensubstanzgehalt zwischen 65 und 77 Gew-% aufweist.

6. Verfahren nach einem der Ansprüche 1 bis 5, dadurch gekennzeichnet, dass der Gehalt des in Anwendung gebrachten Sorbitsirups an D-Sorbit höher als 95% ist.

7. Verfahren nach einem der Ansprüche 1 bis 6, dadurch gekennzeichnet, dass der Gehalt des in Anwendung gebrachten Sorbitsirups an D-Sorbit höher als 99% ist.

8. Verfahren nach einem der Ansprüche 1 bis 7, dadurch gekennzeichnet, dass die innerhalb des bewegten Bettes aus zu überziehenden Kernen herrschende Temperatur bei einem Wert zwischen 50 und 10°C gehalten wird.

9. Verfahren nach irgend einem der Ansprüche 1 bis 7, dadurch gekennzeichnet, dass die innerhalb des bewegten Bettes aus zu überziehenden Kernen hersschende Temperatur bei einem Wert zwischen 40 und 15°C gehalten wird.

10. Verfahren nach irgend einem der Ansprüche 1 bis 9, dadurch gekennzeichnet, dass der auf die Ebene der zu überziehenden Kerne gelangende Sorbit sich auf einem Sättigungsniveau zwischen 0,8 und 1,15 befindet.

11. Anwendung des Verfahrens nach einem der Ansprüche 1 bis 10 beim Dragieren von Kernen "ohne Zucker" auf dem Gebiet der Zuckerwaren und der Pharmazeutika.

## Claims

1. Hard coating process with sorbitol, characterised by the fact that:
— on the one hand, the sorbitol is applied by addition, on a moving bed of cores to be coated, of a syrup having a temperature lower than 100°C and having a concentration of dry matter comprised between 60 and 85% by weight, the content of this syrup in D-sorbitol being higher than 80%,
— on the other hand, the temperature existing in the moving bed of cores to be coated is kept at a value below 55°C, all of these conditions being selected, within the limits indicated, in such a manner that, when the sorbitol syrup arrives in contact with the cores to be coated, that is to say at the temperature maintained in the moving bed, it is at a saturation level comprised between 0.65 and 1.25.

9

2. Process according to claim 1, characterised by the fact that the temperature of the syrup of sorbitol as used is lower than 90°C.

3. Process according to one of claims 1 and 2, characterised by the fact that the temperature of the syrup of sorbitol as used is lower than 70°C.

4. Process according to one of claims 1 to 3, characterised by the fact that the syrup of sorbitol as used has a dry matter content comprised between 62 and 80% by weight.

5. Process according to one of claims 1 to 3, characterised by the fact that the syrup of sorbitol as used has a dry matter content comprised between 65 and 77% by weight.

6. Process according to one of claims 1 to 5, characterised by the fact that the content in D-sorbitol of the syrup of sorbitol as used is higher than 95%.

7. Process according to one of claims 1 to 6, characterised by the fact that the content in D-sorbitol of the syrup of sorbitol as used is higher than 99%.

8. Process according to any one of claims 1 to 7, characterised by the fact that the temperature existing in the moving bed of cores to be coated is kept at a value comprised between 50 and 10°C.

9. Process according to any one of claims 1 to 7, characterised by the fact that the temperature existing in the moving bed of cores to be coated is kept at a value comprised between 40 and 15°C.

10. Process according to any one of claims 1 to 9, characterised by the fact that, when the sorbitol arrives in contact with the cores to be coated, it is at a saturation level comprised between 0.8 and 1.15.

11. Application of the process according to one of claims 1 to 10, to the coating of cores of the "sugarless" type in the field of confectionery and pharmaceutical products.